# EUROPEAN PATENT SPECIFICATION

(11) **EP 0 493 087 B1**
(45) Date of publication and mention of the grant of the patent: **13.03.1996**
(21) Application number: 91311977.2
(22) Date of filing: 23.12.1991
(51) Int. Cl.: C07C 271/22, C07C 269/08

(54) **Process for the crystallisation of N-t-butyloxycarbonyl-3-cyclohexyl-L-alanine methyl ester**
Verfahren zur Kristallisierung von N-t-Butoxycarbonyl-3-cyclohexyl-L-alaninmethylester
Procédé pour la cristallisation de l'ester méthylique de la N-t-butyloxycarbonyl-3-cyclohexyl-L-alanine

(30) Priority: 28.12.1990 JP 418803/90
(43) Date of publication of application: 01.07.1992
(73) Proprietor: Ajinomoto Co., Inc., Tokyo 104 (JP)
(72) Inventor: Kurauchi, Masahiko, Kawasaki Plant, Kawasaki-ku, Kawasaki-shi, Kanagawa-ken (JP); Nakamura, Tohru, Kawasaki Plant, Kawasaki-ku, Kawasaki-shi, Kanagawa-ken (JP)
(74) Representative: Nicholls, Kathryn Margaret

(56) References cited:
- EP-A- 0 189 203
- US-A- 4 948 913
- CHEMICAL ABSTRACTS, vol. 103, no. 23, 9 December 1985, page 713, abstract no. 196 406z, Columbus, Ohio, US; J. BOGER et al, "Renin inhibitors. Syntheses of subnanomolar, competitive, transition-state analog inhibitors containing a novel analog of statine"

## Description

The present invention relates to crystals of N-t-butyloxycarbonyl-3-cyclohexyl-L-alanine methyl ester which is important as an intermediate in the manufacture of pharmaceuticals, and to the production of such crystals.

N-t-butyloxycarbonyl-3-cyclohexyl-L-alanine methyl ester shown by the following formula (1) : 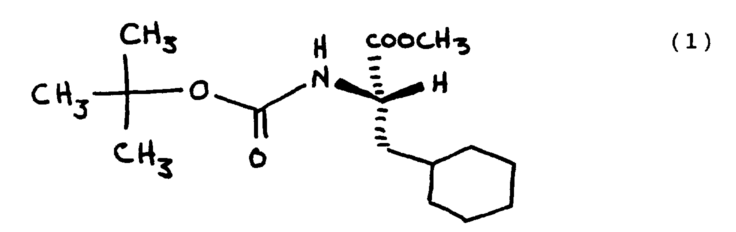 is an important compound as an intermediate of various pharmaceuticals.

This compound can be synthesized by the method disclosed by J. Boger et al. (J. Med. Chem., 28, 1779 (1985)) but N-t-butyloxycarbonyl-3-cyclohexyl-L-alanine methyl ester is obtained as an oily substance. There have been no reports of the compound being obtained in the form of crystals.

It is desirable to produce crystals of N-t-butyloxycarbonyl-3-cyclohexyl-L-alanine methyl ester since the substance in this form would have a higher purity than the oil and thus be better suited to use as an intermediate in the pharmaceutical industry. Furthermore, it would be easier to transport and handle.

The present inventors have made various investigations to solve the problem described above and as a result, have found that by adding an organic solvent to the oily N-t-butyloxycarbonyl-3-cyclohexyl-L-alanine methyl ester, crystals of formula (1) can be obtained.

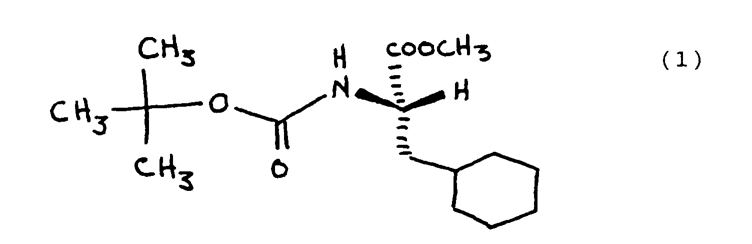

According to a first aspect of the invention there is provided a method for the production of crystalline N-t-butyloxycarbonyl-3-cyclohexyl-L-alanine methyl ester comprising addition of an organic solvent to said ester in oily form. Preferably the resulting mixture of ester and solvent is subsequently cooled, for example from a temperature of above 20°C to below 20°C. Crystals produced previously may be used to seed the mixture of ester and solvent.

The crystals can, for example, be obtained by adding to the oily N-t-butyloxycarbonyl-3-cyclohexyl-L-alanine methyl ester a saturated hydrocarbon solvent for example having from 5 to 9 carbon atoms such as n-hexane, cyclohexane, petroleum ether or petroleum benzine and/or a lower alkanol such as a C₁₋₄ alkanol, eg methanol, ethanol or isopropyl alcohol; of the saturated hydrocarbons n-hexane is preferred; of the lower alcohols methanol is preferred. In some embodiments a mixture of methanol and n-hexane may be employed.

When only a hydrocarbon such as n-hexane is used, up to 50wt%, preferably 25 to 40wt% of hydrocarbon to N-t-butyloxycarbonyl-3cyclohexyl-L-alanine methyl ester is added. When only a lower alkanol such as methanol is used, up to 35wt%, preferably 15 to 25wt% of alkanol is added. When both a hydrocarbon and an alkanol (such as a combination of n-hexane and methanol) are used, up to 40wt%, preferably 3 to 30wt% of the hydrocarbon and up to 25wt%, preferably I to 15wt% of the alkanol is used.

As an alternative to the use of solvent there is provided, according to a second aspect of the present invention, a method for the production of N-t-butyloxycarbonyl-3-cyclohexyl-L-alanine methyl ester of formula(1) in crystalline form, which method comprises adding to the ester in oily form one or more seed crystals according to the first aspect. These crystals may have been previously produced by the use of a solvent by a method according to the first aspect of the invention.

N-t-butyloxycarbonyl-3-cyclohexyl-L-alanine methyl ester can be prepared by reacting, for example, L-phenylalanine methyl ester hydrochloride with di-t-butyl dicarbonate in the presence of, for example, triethylamine and reducing the resulting N-t-butyloxycarbonyl-L-phenyl alanine methyl ester with hydrogen in methanol in the presence of a rhodium catalyst.

### Examples

Embodiments of the present invention are described below by way of example only and with reference to the accompanying figures, of which:

Fig. 1 shows a powder X-ray chart of the crystals prepared in Example 1.

Fig. 2 shows a differential scanning calorimeter (DSC) chart for the crystals prepared in Example 1.

Figs. 3a-d show microscope photos at x40 magnification of the crystals prepared according to Example 3.

Fig. 4 shows a microscope photo at x100 magnification of a crystal prepared in Example 4.

Fig. 5 shows a microscope photo at x40 magnification of crystals prepared in Example 5.

### Example 1

After 13.0 ml (6.8wt%) of n-hexane and 40 ml (2.6wt%) of methanol were added to 126 g of oily N-t-butyloxycarbonyl-3-cyclohexyl-L-alanine methyl ester at room temperature (about 25°C), the mixture was cooled to 4°C while stirring. The precipitated crystals were immediately separated by centrifuging and washed with 7 ml of n-hexane previously cooled. After the obtained crystals were air-dried, the crystals were dried at 30°C overnight under reduced pressure to give 3.7 g of N-t-butyloxycarbonyl-3-cyclohexyl-L-alanine methyl ester as crystals.

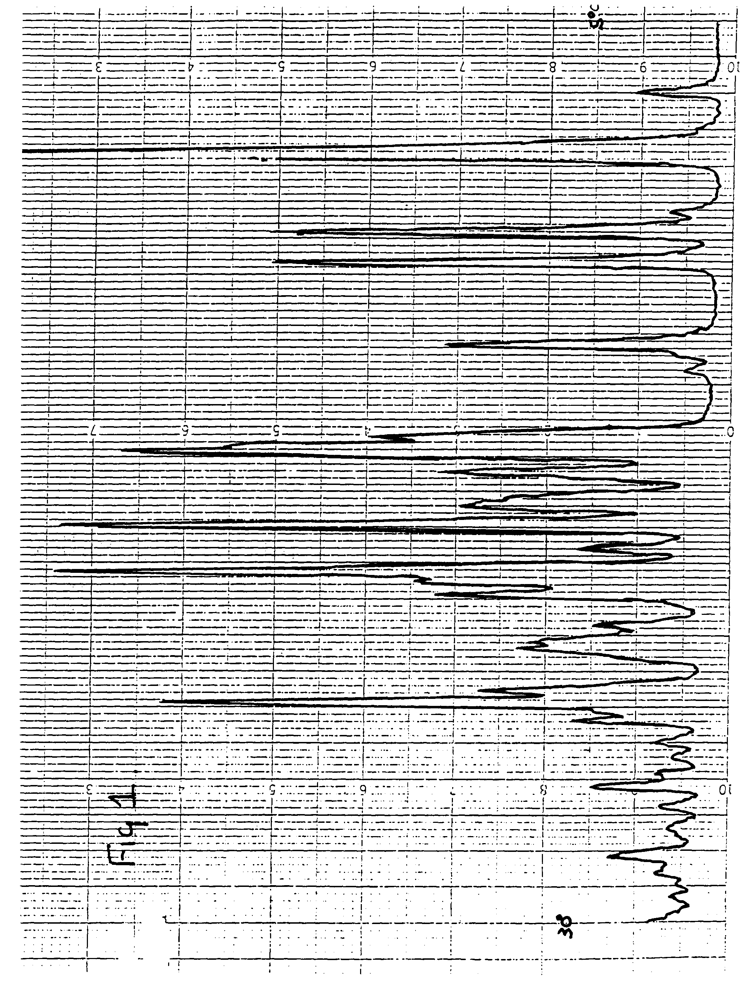

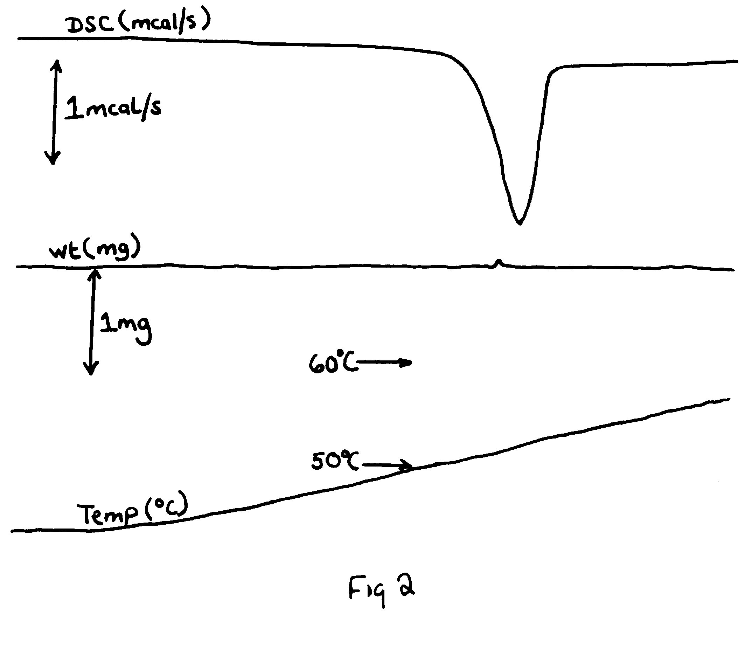

The crystals were subjected to a variety of analytical tests as specified below. The powder X-ray chart of the crystals is attached as Fig. 1 and a DSC chart as Fig. 2. It should be noted that because of the way the DSC was set up the phase change appears to take place at a temperature higher than that measured using a melting point apparatus and specified below.
¹H-NMR ( CDC1₃): 4.87 (d, 1H), 4.32 (d, 1H), 3.72 (s, 3H), 1.81 (d, 1H), 1.66 (m, 6H), 1.43 (s, 9H), 1.33 (m, 1H), 1.18 (m, 3H), 0.92 (m, 2H)
Mass spectrum (FAB): 286(M⁺)
Specific rotary power (20°C, C=1, methanol): -20.0°
Melting point: 48.9 - 49.2°C

### Example 2

After 9.3 ml (9.9wt%) of n-hexane and 4.7 ml (6.2wt%) of methanol were added to 62 g of oily N-t-butyloxycarbonyl-3-cyclohexyl-L-alanine methyl ester at room temperature (about 25°C), the mixture was gradually cooled while stirring. When the temperature of the liquid reached 15°C, 0.1 g of the crystals of N-t-butyloxycarbonyl-3-cyclohexyl-L-alanine methyl ester prepared in Example 1 was added as seed crystals and cooling was further continued while stirring until the temperature reached 4°C. The precipitated crystals were immediately separated by centrifuge and washed with 3 ml of n-hexane previously cooled. After the obtained crystals were air-dried, the crystals were dried at 30°C overnight under reduced pressure to give 28.5 g of N-t-butyloxycarbonyl-3-cyclohexyl-L-alanine methyl ester as crystals.

### Example 3

After 25.0 ml (19.8wt%) of methanol was added to 100g of oily N-t-butyloxycarbonyl-3-cyclohexyl-L-alanine methyl ester at room temperature (about 25°C), the mixture was gradually cooled while stirring. When the temperature of liquid reached 13°C, 1.0g of the crystals of N-t-butyloxycarbonyl-3-cyclohexyl-L-alanine methyl ester prepared in Example 1 was added as seed crystals and cooling was continued to 12°C while stirring. The precipitated crystals were immediately separated by centrifuge and washed with 5 ml of methanol previously cooled. After the obtained crystals were air-dried, the crystals were dried at 30°C overnight under reduced pressure to give 71.2g of N-t-butyloxycarbonyl-3-cyclohexyl-L-alanine methyl ester as crystals. Microscope photos of crystals formed are shown at Figs. 3a - d.

### Example 4

After 50.0 ml (33.0wt%) of n-hexane was added to 100g of oily N-t-butyloxycarbonyl-3-cyclohexyl-L-alanine methyl ester at room temperature (about 25°C), the mixture was gradually cooled while stirring. When the temperature of liquid reached 22°C, 1.0 g of the crystals of N-t-butyloxycarbonyl-3-cyclohexyl-L-alanine methyl ester prepared in Example 1 was added as seed crystals and cooling was continued to 20°C while stirring. The precipitated crystals were immediately separated by a centrifugal machine and washed with 5 ml of n-hexane previously cooled. After the obtained crystals were airdried, the crystals were dried at 30°C overnight under reduced pressure to give 79.9g of N-t-butyloxycarbonyl-3-cyclohexyl-L-alanine methyl ester as crystals.

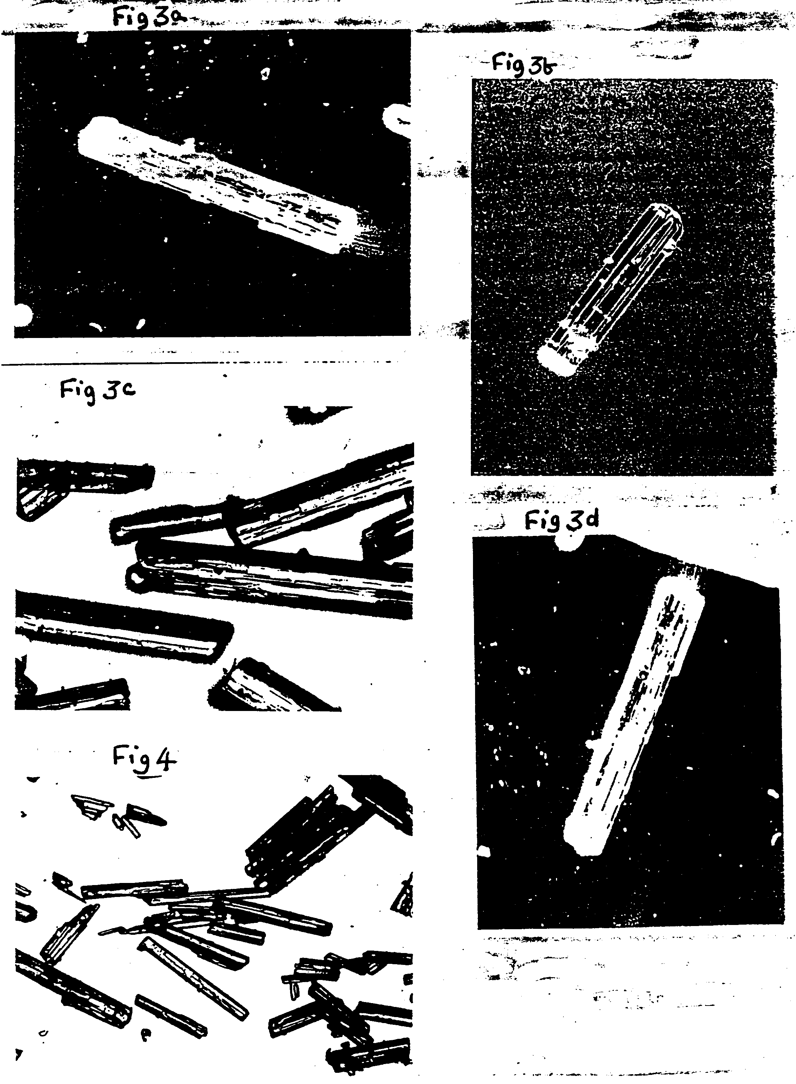

Crystals formed in this way are shown in Fig. 4.

### Example 5

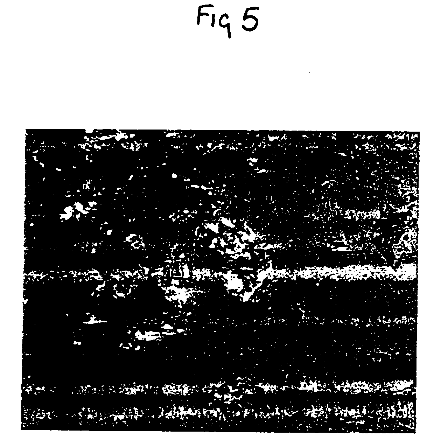

After 25.0 ml (19.8wt%) of methanol was added to 100g of oily N-t-butyloxycarbonyl-3-cyclohexyl-L-alanine methyl ester at room temperature (about 25°C), the mixture was heated to 40°C while stirring. When the temperature of liquid reached 40°C, methanol was evaporated under reduced pressure. Oily N-t-butyloxycarbonyl-3-cyclohexyl-L-alanine methyl ester was again obtained. 1.0g of the crystals of N-t-butyloxycarbonyl-3-cyclohexyl-L-alanine methyl ester prepared in Example 1 was added to the oil as seed crystals and cooling was gradually continued to 15°C while stirring. The precipitated crystals were immediately separated by a centrifugal machine and washed with 5ml of methanol previously cooled. After the obtained crystals were air-dried, the crystals were dried at 30°C overnight under reduced pressure to give 84.3g of N-t-butyloxycarbonyl-3-cyclohexyl-L-alanine methyl ester as crystals. The powder X-ray chart for the crystals formed in this way resembled that shown in Fig. 1 for the crystals of Example 1. A microscope photo of the crystals is shown at Fig. 5.

## Claims

1. A method for the production of crystals of N-t-butyloxycarbonyl-3-cyclohexyl-L-alanine methyl ester of formula (1) comprising addition of a lower alkanol and/or a saturated hydrocarbon to said ester in oily form

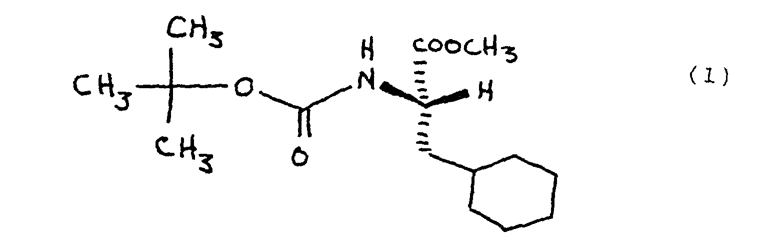

2. A method according to claim 1 wherein a hydrocarbon is used as solvent in an amount of from 25 to 40 weight% of hydrocarbon to N-t-butyloxycarbonyl-3-cyclohexyl-L-alanine methyl ester.

3. A method according to claim 1 wherein a lower alkanol is used as solvent in an amount of from 15 to 25 weight% of alkanol to N-t-butyloxycarbonyl-3-cyclohexyl-L-alanine methyl ester.

4. A method according to claim 1 wherein a mixture of hydrocarbon and alkanol are used; the hydrocarbon being used in an amount of from 3 to 30wt% and the alkanol in an amount of from 1 to 15wt% to N-t-butyloxycarbonyl-3-cyclohexyl-L-alanine methyl ester.

5. A method according to any preceding claim wherein the lower alkanol is selected from methanol, ethanol and isopropanol, and the hydrocarbon is selected from n-hexane, cyclohexane, petroleum ether and petroleum benzine.

6. A method according to claim 5 wherein the solvent is methanol and/or n-hexane.

7. A method according to any preceding claim wherein the mixture formed by addition of a solvent to said ester in oily form is subsequently cooled.

8. A method according to claim 7 wherein solvent is added to the oil at a temperature of 20°C or above, the resulting mixture then being cooled to below 20°C.

9. A method according to any preceding claim wherein a seed crystal as obtainable by the method of any one of said claims is added to the mixture formed by addition of solvent to the ester in oily form, so as to promote crystallisation.

10. A method according to any one of the preceding claims, further comprising using the crystals as obtained by the method of any one of said claims to seed further ester in oily form, thereby to produce crystals.

11. A method according to any one of claims 1 to 10 further comprising using the crystals as obtained by the method of any one of said claims as an intermediate in the manufacture of a pharmaceutical.

## Patentansprüche

1. Verfahren zur Herstellung von Kristallen aus N-tert.- Butyloxycarbonyl-3-cyclohexyl-L-alaninmethylester der Formel (1), das die Zugabe eines niederen Alkanols und/oder eines gesättigten Kohlenwasserstoffs zu dem Ester, der in ölartiger Form vorliegt, 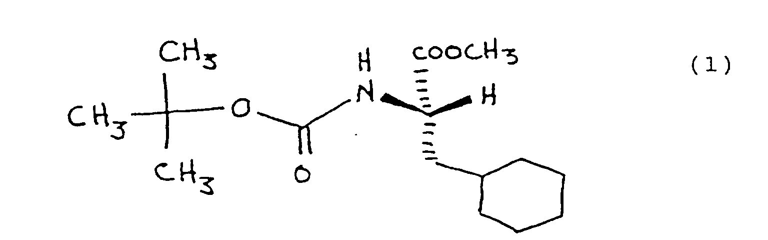 umfaßt.

2. Verfahren gemäß Anspruch 1, wobei ein Kohlenwasserstoff als Lösungsmittel in einer Menge von 25 bis 40 Gew.-% bezogen auf N-tert.-Butyloxycarbonyl-3-cyclohexyl-L-alaninmethylester verwendet wird.

3. Verfahren gemäß Anspruch 1, wobei ein niederes Alkanol als Lösungsmittel in einer Menge von 15 bis 25 Gew.-% bezogen auf N-tert.-Butyloxycarbonyl-3-cyclohexyl-L-alaninmethylester verwendet wird.

4. Verfahren gemäß Anspruch 1, wobei ein Gemisch aus Kohlenwasserstoff und Alkanol verwendet wird, wobei der Kohlenwasserstoff in einer Menge von 3 bis 30 Gew.-% und das Alkanol in einer Menge von 1 bis 15 Gew.-% bezogen auf N-tert.- Butyloxycarbonyl-3-cyclohexyl-L-alaninmethylester verwendet wird.

5. Verfahren gemäß einem der vorhergehenden Ansprüche, wobei das niedere Alkanol unter Methanol, Ethanol und Isopropanol und der Kohlenwasserstoff unter n-Hexan, Cyclohexan, Petrolether und Leichtbenzin ausgewählt wird.

6. Verfahren gemäß Anspruch 5, wobei das Lösungsmittel Methanol und/oder n-Hexan ist.

7. Verfahren gemäß einem der vorhergehenden Ansprüche, wobei das Gemisch, das durch Zugabe eines Lösungsmittels zu dem Ester, der in ölartiger Form vorliegt, gebildet wird, anschließend gekühlt wird.

8. Verfahren gemäß Anspruch 7, wobei das Lösungsmittel bei einer Temperatur von 20°C oder darüber dem Öl zugegeben wird, und anschließend das resultierende Gemisch auf unter 20°C abgekühlt wird.

9. Verfahren gemäß einem der vorhergehenden Ansprüche, wobei zur Beschleunigung der Kristallisation ein gemäß dem Verfahren nach einem der genannten Ansprüche erhältlicher Impfkristall zu dem durch Zugabe eines Lösungsmittels zu dem in ölartiger Form vorliegenden Ester gebildeten Gemisch gegeben wird.

10. Verfahren gemäß einem der vorhergehenden Ansprüche, das ferner die Verwendung der gemäß dem Verfahren nach einem der genannten Ansprüche erhaltenen Kristalle zum weiteren Animpfen des in ölartiger Form vorliegenden Esters umfaßt, wobei Kristalle erzeugt werden.

11. Verfahren gemäß einem der Ansprüche 1 bis 10, das ferner die Verwendung der gemäß dem Verfahren nach einem der genannten Ansprüche erhaltenen Kristalle als Zwischenprodukt bei der Herstellung eines Arzneimittels umfaßt.

## Revendications

1. Méthode de production de cristaux d'ester de méthyle de la N-t-butyloxycarbonyl-3-cyclohexyl-L-alanine de formule (1) constituée par l'addition d'un alcanol inférieur et/ou d'un hydrocarbure saturé audit ester sous forme huileuse

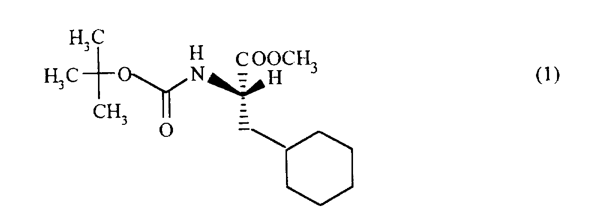

2. Méthode selon la revendication 1, dans laquelle on utilise un hydrocarbure en tant que solvant en une quantité allant de 25 à 40 % d'hydrocarbure en masse par rapport à l'ester de méthyle de la N-t-butyloxycarbonyl-3-cyclohexyl-L-alanine.

3. Méthode selon la revendication 1, dans laquelle on utilise un alcanol inférieur en tant que solvant en une quantité allant de 15 à 25 % d'alcanol en masse par rapport à l'ester de méthyle de la N-t-butyloxycarbonyl-3-cyclohexyl-L-alanine.

4. Méthode selon la revendication 1, dans laquelle on utilise un mélange d'hydrocarbure et d'alcanol, l'hydrocarbure étant utilisé en une quantité allant de 3 à 30 % en masse et l'alcanol en une quantité allant de 1 à 15 % en masse par rapport à l'ester de méthyle de la N-t-butyloxycarbonyl-3-cyclohexyl-L-alanine.

5. Méthode selon une quelconque des revendications qui précédent, dans laquelle l'alcanol inférieur est choisi parmi le méthanol, l'éthanol et l'isopropanol et l'hydrocarbure est choisi parmi le n-hexane, le cyclohexane, l'éther de pétrole et l'essence de pétrole.

6. Méthode selon la revendication 5, dans laquelle le solvant est le méthanol et/ou le n-hexane.

7. Méthode selon une quelconque des revendications qui précèdent, dans laquelle le mélange formé par l'addition d'un solvant audit ester sous forme huileuse est ensuite refroidi.

8. Méthode selon la revendication 7, dans laquelle le solvant est ajouté à l'huile à une température de 20°C ou plus, le mélange résultant étant ensuite refroidi au-dessous de 20°C.

9. Méthode selon une quelconque des revendications qui précèdent, dans laquelle un germe cristallin tel qu'il peut être obtenu par la méthode d'une quelconque desdites revendications est ajouté au mélange formé par l'addition d'un solvant à l'ester à l'état huileux, de façon à favoriser la cristallisation.

10. Méthode selon une quelconque des revendications qui précèdent, comprenant en outre l'utilisation des cristaux tels qu'ils sont obtenus par la méthode d'une quelconque desdites revendications pour ensemencer une quantité supplémentaire d'ester sous forme huileuse, et ainsi produire des cristaux.

11. Méthode selon une quelconque des revendications 1 à 10 comprenant en outre l'utilisation des cristaux tels qu'ils sont obtenus par la méthode d'une quelconque desdites revendications en tant qu'intermédiaire pour la fabrication d'un produit pharmaceutique.
